# EUROPEAN PATENT SPECIFICATION

(11) **EP 3 843 639 B1**
(45) Date of publication and mention of the grant of the patent: **13.03.2024**
(21) Application number: 19783430.2
(22) Date of filing: 28.08.2019
(51) Int. Cl.: A61B 8/12, A61B 8/14, A61B 8/00, G01S 15/89

(54) **IMAGING PLANE CONTROL AND DISPLAY FOR INTRALUMINAL ULTRASOUND, AND ASSOCIATED DEVICES, SYSTEMS, AND METHODS**
STEUERUNG UND ANZEIGE DER BILDGEBUNGSEBENE FÜR INTRALUMINALEN ULTRASCHALL SOWIE ZUGEHÖRIGE VORRICHTUNGEN, SYSTEME UND VERFAHREN
COMMANDE ET AFFICHAGE DE PLAN D'IMAGERIE POUR SYSTÈME ULTRASONORE INTRALUMINAL, DISPOSITIFS ET PROCÉDÉS ASSOCIÉS

(30) Priority: 29.08.2018 US 201862724179 P
(43) Date of publication of application: 07.07.2021
(73) Proprietor: Koninklijke Philips N.V., 5656 AG Eindhoven (NL)
(72) Inventor: CLARK, David, Wesley, 5656 AE Eindhoven (NL); DASARI, Ramanjini, 5656 AE Eindhoven (NL); YENIN, Ingrid, 5656 AE Eindhoven (NL); JIANG, Wei, 5656 AE Eindhoven (NL); MANIAN, Suresh, 5656 AE Eindhoven (NL)
(74) Representative: Philips Intellectual Property & Standards
(86) International application number: PCT/EP2019/073010
(87) International publication number: WO 2020/043795

(56) References cited:
- WO-A1-2015/068073
- WO-A1-2018/065282
- US-A1- 2010 249 589
- US-A1- 2011 071 395

## Description

### TECHNICAL FIELD

The present disclosure relates generally to user interfaces for intraluminal imaging systems, and in particular, for intra-cardiac echocardiography (ICE) systems. The user interfaces may be configured to measure and display the angles of imaging planes with respect to the axial direction of the aperture of the ICE system. The user interfaces may also be configured to receive input to automatically switch between various angles of imaging planes. The user interfaces may also be configured to toggle between optimizations for different imaging setting, such as biological tissue and tools.

### BACKGROUND

Diagnostic and therapeutic ultrasound catheters have been designed for use inside many areas of the human body. In the cardiovascular system, a common diagnostic ultrasound method is intraluminal ultrasound imaging with intra-cardiac echocardiography (ICE) being a specific example of intraluminal imaging. Typically, a single rotating transducer or an array of transducer elements is used to transmit ultrasound at the tips of the catheters. The same transducers (or separate transducers) are used to receive echoes from the tissue. A signal generated from the echoes is transferred to a console which allows for the processing, storing, display, or manipulation of the ultrasound-related data.

Intraluminal ultrasound catheters are typically used in the large and small blood vessels (arteries or veins) of the body and are almost always delivered over a guidewire having a flexible tip. Intraluminal imaging catheters such as ICE catheters are usually used to image heart and surrounding structures, for example, to guide and facilitate medical procedures, such as transseptal lumen punctures, left atrial appendage closures, atrial fibrillation ablation, and valve repairs. Commercially-available ICE imaging catheters are not designed to be delivered over a guidewire, but instead have distal ends which can be articulated by a steering mechanism located in a handle at the proximal end of the catheter. For example, an intraluminal imaging catheter such as an ICE catheter may be inserted through the femoral or jugular vein when accessing the anatomy, and steered in the heart to acquire images necessary to the safety of the medical procedures.

An ICE catheter typically includes imaging transducers for ultrasound imaging that generates and receives acoustic energy. The imaging core may include a lined array of transducer elements or transducer elements arranged in any suitable configuration. The imaging core is encased in an imaging assembly located at a furthest distal tip of the catheter. The imaging assembly is covered with acoustic adhesive materials. An electrical cable is soldered to the imaging core and extends through the core of the body of the catheter. The electrical cable may carry control signals and echo signals to facilitate imaging of the heart anatomy. The assembly may provide rotational, 2-way, or 4-way steering mechanisms such that anterior, posterior, left, and/or right views of the heart anatomy may be imaged.

ICE imaging transducers with 1D arrays are well known (e.g. Siemens Acunav, St. Jude ViewFlex). These transducers are introduced to the interior of the heart via a blood vessel by means of a catheter, and can acquire images only in a fixed plane aligned with the axis of the catheter. ICE transducers with 2D arrays are being developed which can acquire 2D images in a variety of planes, simultaneous 2D images in several planes (x-plane), or 3D images. The 2D arrays may be rectangular in shape with an asymmetrical aperture, in which the aperture is longer in one direction and shorter in the other.

US 2015/068073 discloses an external imaging probe, for tracking a catheter or other interventional tool. US 2011/0071395 discloses a transesophageal probe. WO 2018/065282 relates to an intraluminal probe. It recognizes that different imaging planes have different imaging resolution, and it thereby allows the imaging angle to be adjusted.

### SUMMARY

The inventors recognized that ICE transducers with 2D arrays, while allowing for a larger variety of imaging planes and enabling 3D imaging, may also cause resolution issues due to the asymmetric aperture configurations. In particular, the asymmetric nature of the 2D imaging aperture may cause different imaging resolutions in images at different imaging planes along respective angles. For example, the imaging aperture may acquire a first image in a longitudinal direction (0°) and a second image in a transverse direction (90°) with respect to the imaging aperture. In this case, the first and second images may have different imaging resolutions as a result of the different dimensions of the aperture in the longitudinal and transverse directions. The variances in resolution from imaging at different angles may not be readily apparent to an operator, causing correction of the resolution and degraded image quality a difficult task to even the most skilled clinicians. Furthermore, an operator may need to view imaging data in different optimization settings, such as to focus on different parts of a vessel. Changing between these optimization setting may require manual changing parameters of imaging data. This may be time consuming and difficult to accomplish.

The present invention provides systems that account for the issues associated with the asymmetrical aperture of such 2D arrays, thereby allowing users to take advantage of their 2D and 3D imaging without being impeded resolution and workflow issues caused by the asymmetrical aperture. In particular, user interfaces for intravascular imaging systems are provided to help an operator understand and control the positions of imaging planes, such as showing the angular positions of imaging planes with respect to an imaging aperture, automatically switching to imaging planes with better resolution, and toggling between various imaging optimizations. The invention is defined in the independent claims 1 and 14.

In one embodiment, an ultrasound imaging system according to claim 1 is provided.

The processor is further configured to compare the first angular position to a threshold angular position above which compromised imaging performance occurs. The visual representation may be a comparison of the first angular position and the threshold angular position. The visual representation may include an alert if the first angular position exceeds the threshold angular position. The visual representation may include a color representing a difference between the first angular position and the threshold angular position.

In some embodiments, the processor is configured to optimize the imaging data based on a plurality of imaging settings, wherein the processor is configured to receive a first selection of the plurality of imaging settings from a user and display the imaging data with optimized imaging data corresponding to the selected imaging setting on the display device. The optimizations may include at least one of gain, dynamic range, gray map, spatial smoothing, beamforming, frequency, or chroma. The processor may be configured to receive the first selection of the plurality of imaging settings from the display device.

In some embodiments, the processor is configured to display optimized imaging data corresponding to a first and a second selection of the plurality of imaging settings on the display device, wherein the display device is configured to toggle between the optimized imaging data corresponding to the first and second selections of the plurality of imaging settings. The processor may be further configured to: receive second imaging data from the transducer array along a second imaging plane at a second angular position with respect to the axial direction of the aperture; and output the second imaging data to the display device.

The display device may be configured to show the first and second imaging data simultaneously in a side by side display. The processor may be further configured to: receive third imaging data from the transducer array along a third imaging plane at a third angular position with respect to the axial direction of the aperture; receive fourth imaging data from the transducer array along a fourth imaging plane at a fourth angular position with respect to the axial direction of the aperture; and output the third and fourth imaging data to the display device.

The processor may be configured to receive a second selection to automatically change the display of the first and second imaging data to the third and fourth imaging data. The display device may be configured to toggle between the imaging data corresponding to the first and second selections. The third angular position may be +45 degrees with respect to the axial direction of the aperture, wherein the fourth angular position may be -45 degrees with respect to the axial direction of the aperture. In some embodiments, the third angular position is 0 degrees with respect to the axial direction of the aperture, wherein the fourth angular position is +60 degrees with respect to the axial direction of the aperture.

An exemplary ultrasound imaging method is also described, comprising: receiving, at a processor in communication with an intraluminal ultrasound device positioned within a body lumen of patient, first imaging data from a transducer array of the intraluminal ultrasound device, the transducer array comprising an aperture and obtaining imaging data along one or more imaging planes, wherein the first imaging data is obtained along a first imaging plane at a first angular position with respect to an axial direction of the aperture; outputting, to a display device in communication with the processor, the first imaging data; and outputting, to the display device, a visual representation of the first angular position of the first imaging plane with respect to the axial direction of the aperture.

A method may further include comparing, at the processor, the first angular position to a threshold angular position above which compromised imaging performance occurs, wherein the visual representation comprises an alert if the first angular position exceeds the threshold angular position. The method may further include: receiving a first selection of a plurality of imaging settings from a user; optimizing the imaging data based on the first selection of plurality of imaging settings, wherein outputting the first imaging data comprising displaying, on the display device, optimized imaging data corresponding to the first selection of the plurality of imaging settings. The method may also include receiving, at the processor, second imaging data from the transducer array along a second imaging plane at a second angular position with respect to the axial direction of the aperture; and outputting, to the display device, the second imaging data in a side by side display simultaneously with the first imaging data.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a schematic diagram of an intraluminal imaging system according to aspects of the present disclosure.
Fig. 2 is a perspective view of an imaging assembly according to aspects of the present disclosure.
Fig. 3 is a top view of a tip member according to aspects of the present disclosure.
Fig. 4A is a schematic diagram illustrating two imaging planes extending at oblique angles relative to a longitudinal axis of an asymmetric aperture of an imaging array according to aspects of the present disclosure.
Fig. 4B is a schematic diagram illustrating two imaging planes extending coplanar with and at a right angle relative to a longitudinal axis of an asymmetric aperture of an imaging array according to aspects of the present disclosure.
Fig. 5 shows ultrasound images at 0 degree and 90 degree planes relative to an asymmetric aperture of an imaging array according to aspects of the present disclosure.
Fig. 6 shows ultrasound images at 0 degree and +45 degree planes relative to an asymmetric aperture of an imaging array according to aspects of the present disclosure.
Fig. 7 shows ultrasound images at ±45 degree planes relative to an asymmetric aperture of an imaging array according to aspects of the present disclosure.
Fig. 8 shows ultrasound images at ±60 degree planes relative to an asymmetric aperture of an imaging array according to aspects of the present disclosure.
Fig. 9 shows ultrasound images at 0 degree and -110 degree planes relative to an asymmetric aperture of an imaging array according to aspects of the present disclosure.
Fig. 10 shows an ultrasound image at a 0 degree plane relative to an asymmetric aperture of an imaging array according to aspects of the present disclosure.
Fig. 11 shows an ultrasound image at a -61 degree plane relative to an asymmetric aperture of an imaging array according to aspects of the present disclosure.
Fig. 12 shows an ultrasound image at a 61 degree plane relative to an asymmetric aperture of an imaging array according to aspects of the present disclosure.
Fig. 13 shows a user interface for controlling imaging parameters according to aspects of the present invention.
Fig. 14 shows another user interface for controlling imaging parameters according to aspects of the present invention.
Fig. 15 is a flow diagram of a method of performing intraluminal imaging with an intraluminal device according to aspects of the disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the ICE system is described in terms of intraluminal imaging, it is understood that it is not intended to be limited to this application. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

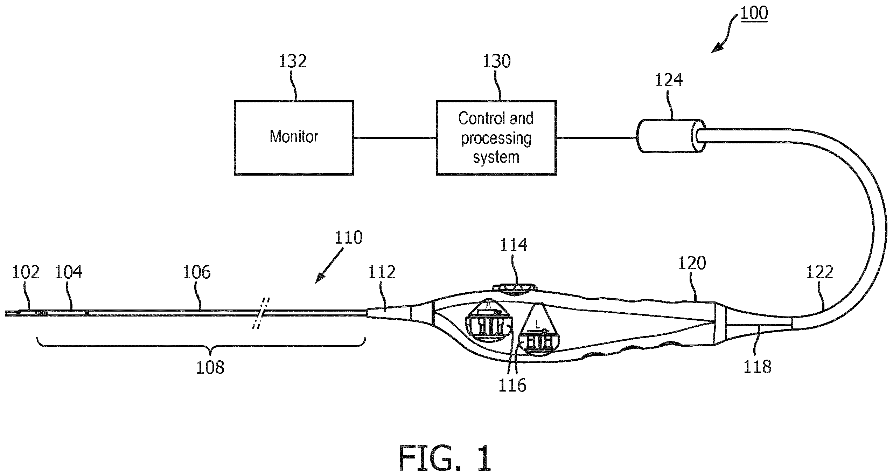

Fig. 1 is a schematic diagram of an ultrasound imaging system 100 according to embodiments of the present disclosure. The system 100 may include an ultrasound imaging device 110, a connector 124, a control and processing system 130, such as a console and/or a computer, and a monitor 132. The system 100 can be an intraluminal imaging system, including an intraluminal imaging device 110. The device 110 can be a catheter, guide wire, or guide catheter. The intraluminal imaging device 110 includes an imaging assembly 102 at the tip of a flexible elongate member 108, and a handle 120. The flexible elongate member 108 includes a distal portion 104 and a proximal portion 106. The distal end of the distal portion 104 is attached to the imaging assembly 102. The proximal end of the proximal portion 106 is attached to the handle 120 for example, by a resilient strain reliever 112, for manipulation of the intraluminal imaging device 110 and manual control of the intraluminal imaging device 110. The imaging assembly 102 can include an imaging core with ultrasound transducer elements and associated circuitry. The handle 120 can include actuators 116, a clutch 114, and other steering control components for steering the intraluminal imaging device 110, such as deflecting the imaging assembly 102 and the distal portion 104, as described in greater details herein.

The handle 120 is connected to the connector 124 via another strain reliever 118 and a connection cable 122. The connector 124 may be configured in any suitable configurations to interconnect with the control and processing system 130 and the monitor 132 for processing, storing, analyzing, manipulating, and displaying data obtained from signals generated by the imaging core at the imaging assembly 102. The control and processing system 130 can include one or more processors, memory, one or more input devices, such as keyboards and any suitable command control interface device. The control and processing system 130 can be operable to facilitate the features of the intraluminal imaging system 100 described herein. For example, the processor can execute computer readable instructions stored on the non-transitory tangible computer readable medium. The monitor 132 can be any suitable display device, such as liquid-crystal display (LCD) panel or the like.

In operation, a physician or a clinician advances the flexible elongate member 108 into a vessel within a heart anatomy. The physician or clinician can steer the flexible elongate member 108 to a position near the area of interest to be imaged by controlling the actuators 116 and the clutch 114 on the handle 120. For example, one actuator 116 may deflect the imaging assembly 102 and the distal portion 104 in a left-right plane and the other actuator 116 may deflect the imaging assembly 102 and the distal portion 104 in an anterior-posterior plane, as discussed in greater details herein. The clutch 114 provides a locking mechanism to lock the positions of the actuators 116 and in turn the deflection of the flexible elongate member while imaging the area of interest.

The imaging process may include activating the ultrasound transducer elements on the imaging assembly 102 to produce ultrasonic energy. A portion of the ultrasonic energy is reflected by the area of interest and the surrounding anatomy, and the ultrasound echo signals are received by the ultrasound transducer elements. The connector 124 transfers the received echo signals to the control and processing system 130 where the ultrasound image is reconstructed and displayed on the monitor 132. In some embodiments, the processing system 130 can control the activation of the ultrasound transducer elements and the reception of the echo signals. In some embodiments, the control and processing system 130 and the monitor 132 may be part of the same system.

The system 100 may be utilized in a variety of applications such as transseptal lumen punctures, left atrial appendage closures, atrial fibrillation ablation, and valve repairs and can be used to image vessels and structures within a living body. Although the system 100 is described in the context of intraluminal imaging procedures, the system 100 is suitable for use with any catheterization procedure, e.g., ICE. In addition, the imaging assembly 102 may include any suitable physiological sensor or component for diagnostic, treatment, and/or therapy. For example, the imaging assembly can include an imaging component, an ablation component, a cutting component, a morcellation component, a pressure-sensing component, a flow-sensing component, a temperature-sensing component, and/or combinations thereof.

In some embodiment, the intraluminal imaging device 110 includes a flexible elongate member108 that can be positioned within a vessel. The flexible elongate member 108 having a distal portion 104 and a proximal portion 106. The intraluminal imaging device 110 includes an imaging assembly 102 that is mounted within the distal portion 104 of the flexible elongate member 108.

In some embodiments, the intraluminal imaging system 100 is used for generating 2D and 3D images. In some examples, the intraluminal imaging system 100 is used for generating simultaneous x-plane images at two different viewing directions, typically perpendicular to each other. X-plane may also be generalized to more than two images. The intraluminal imaging system 100 may also be configured to display imaging angles of x-plane images, provide options to automatically adjust the angles of imaging angles, and provide options to switch between various imaging optimizations.

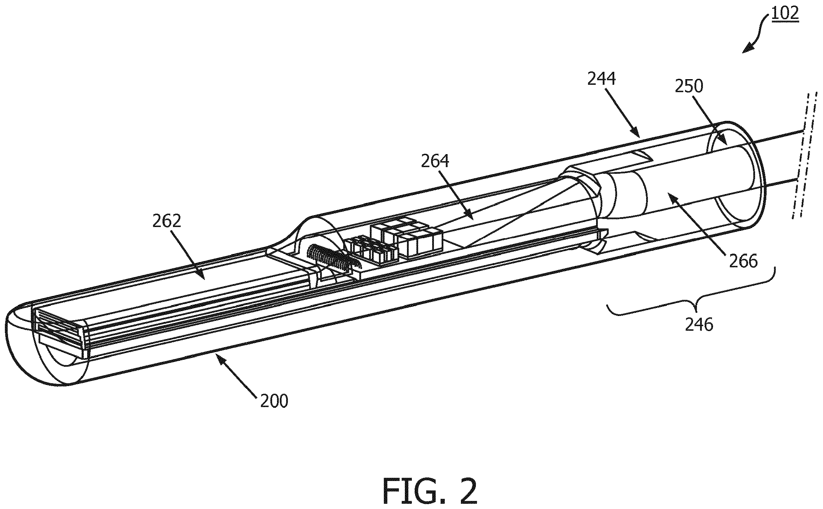

Fig. 2 is a perspective view of the imaging assembly 102 positioned for coupling according to embodiments of the present disclosure. The imaging assembly 102 is illustrated with the imaging core 262 in position within the tip member 200. The imaging core 262 is coupled to the electrical cable 266 via the electrical interconnection 264. The electrical cable 266 extends through the alignment portion 244 and the interface portion 246 of the inner cavity 250. The electrical cable 266 can further extend through the flexible elongate member 108 as shown in Fig. 1.

The configuration and structure of the tip member 200 described above provide several benefits such as safe and easy delivery for catheterization, improved tensile strength for steering or navigation, consistent or automatic alignment, and improved image quality. For example, the outer geometry of the tip member 200 is configured to provide smooth surfaces and smooth edges with small radii. The smooth edges reduce friction when the tip member 200 traverses a vessel during insertion. The smooth surfaces prevent tears and/or damages to tissue structures during the insertion. In addition, the smooth edges and smooth surfaces can facilitate crossing of a septum or other anatomical feature during a catheterization procedure. The material type and the wall thickness of the tip member 200 are selected to minimize acoustic distortion, attenuation, and/or reflection. The internal geometry of the tip member 200 is configured to facilitate alignment during manufacturing. The tip member 200 can also include other features, for example, a guidewire lumen, holes, or other geometry to accommodate additional devices or features such as pressure sensors, drug delivery mechanisms, and/or any suitable interventional features.

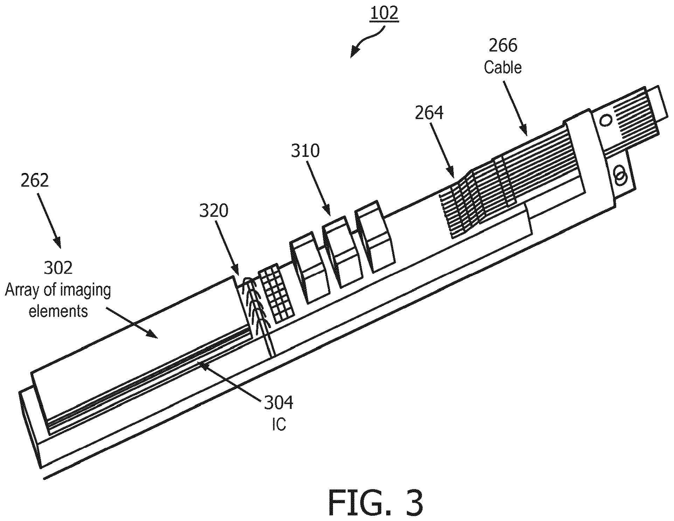

Fig. 3 is a top view of an imaging assembly 102 according to embodiments of the present disclosure. The imaging assembly 102 is illustrated with the imaging core 262 having an array of acoustic elements 302 and micro-beamformer IC 304 coupled to the array of acoustic elements 302. The imaging assembly 102 also shows the electrical cable 266 coupled to the electrical interconnection 264. In some examples, the electrical cable 266 is coupled through an interposer 310 to the micro-beamformer IC 304. In some examples the interposer 310 is connected to the micro-beamformer IC 304 through wire bonding 320. In some examples, the imaging assembly 102 is configured such that the electrical cable 266 is directly coupled to the micro-beamformer IC 304.

In some examples, imaging assembly 102 includes an array of acoustic elements 302 in the form of an array of more than 800 acoustic elements. In this regard, the acoustic elements 302 may be arranged in a 2-dimensional array having a length greater than width such that more acoustic elements 302 extend along the length of the array than across the width. As a result, the array of acoustic elements 302 may have an asymmetrical aperture. In some embodiments, the array of acoustic elements 302 is an array of ultrasound imaging transducers that are directly flip-chip mounted to the micro-beamformer IC 304. The transmitters and receivers of the ultrasound imaging transducers are on the micro-beamformer IC 304 and are directly attached to the transducers. In some examples, mass termination of the acoustic elements is done at the micro-beamformer IC 304.

In some embodiments, the micro-beamforming IC 304 lies directly underneath the array of acoustic elements 302 and is electrically connected to them. The array acoustic elements 302 may be piezoelectric or micromachined ultrasonic transducer (MUT) elements. Piezoelectric elements typically would be attached to the IC by flip-chip mounting an assembly of acoustic layers and sawing into individual elements. MUT elements may be flip-chip mounted as a unit or grown directly on top of the micro-beamforming IC 304. In some examples, the cable bundle may be terminated directly to the micro-beamforming IC 304, or may be terminated to an interposer 310 of suitable material such as a rigid or flexible printed circuit assembly. The interposer 310 may then be connected to the micro-beamforming IC 304 via any suitable means such as wire bonding 320.

In some examples, the micro-beamformer integrated circuit (IC) 304 can control the array of acoustic elements 302 and can perform beam forming for the array of acoustic elements 302. The micro-beamformer integrated IC 304 may be controlled by a processor, such as the control and processing system 130.

In some embodiments, the electrical cable 266 further includes one or more power lines for feeding power to the micro-beamformer IC 304, one or more control lines for communicating control signals to the micro-beamformer IC 304, and one or more signal lines for transferring imaging signals.

In some embodiments, the array of acoustic elements 302 is a two dimensional array. In some examples, not falling within the scope of the invention, the array with acoustic elements 302 is symmetric such that it has equal number of rows of acoustic elements and columns of acoustic elements. The array with acoustic elements 302 is asymmetric such that it has different number of rows of acoustic elements and columns of acoustic elements.

In some embodiments, the delay elements in use consist of a number of repeated elements, and the number of these elements determines the maximum available delay. Since the acoustic array may be flip-chip mounted to the micro-beamformer IC 304, all of the processing, including the delay, for any given element can reside in the area occupied by that one element.

In some examples, a plurality of imaging signals, received by the array of acoustic elements, are beam formed. Each of the plurality of imaging signals is associated with an imaging plane between the first plane and the second plane. A 3D volume image is generated from the plurality of imaging signals such that the 3D image corresponds to a volume image between the first plane and the second plane.

Aspects of the present disclosure can include features similar to those described in U.S. Provisional Application No. 62/403,431, filed 10-03-2016.

The imaging element 302 can include one or more acoustic elements. For example, a plurality of elements can be arranged in an array. For example, an ultrasound transducer array can include any suitable number of individual acoustic elements 302 between 2 acoustic elements and 5000 acoustic elements, including values such as 2 acoustic elements, 4 acoustic elements, 36 acoustic elements, 64 acoustic elements, 128 acoustic elements, 500 acoustic elements, 812 acoustic elements, and/or other values both larger and smaller. The ultrasound transducer array with acoustic elements 302 can be any suitable configuration, such as phased array including a planar array, a curved array, etc. For example, the ultrasound transducer array with acoustic elements 302 can be a one-dimensional array, l.x-dimensional array, such as a 1.5-dimensional array, or a two-dimensional array, in some instances. In that regard, the ultrasound transducer array can be configured obtain one-dimensional, two-dimensional, and/or three-dimensional images of the anatomy of the patient. The ultrasound transducer array with acoustic elements 302 can be a matrix array, including one or more segments of ultrasound elements (e.g., one or more rows, one or more columns, and/or one or more orientations) that can be uniformly or independently controlled and activated. The ultrasound imaging element 302 can include any suitable transducer type, including a piezoelectric micromachined ultrasound transducer (PMUT), capacitive micromachined ultrasonic transducer (CMUT), single crystal, lead zirconate titanate (PZT), PZT composite, other suitable transducer type, and/or combinations thereof.

The flexible elongate member 108 (e.g., a distal portion 104) of the ultrasound imaging device 110 is sized and shaped, structurally arranged, and/or otherwise configured to be positioned within a body lumen of the patient. The body lumen may represent fluid filled or surrounded structures, both natural and man-made. The body lumen may be within a body of a patient. The body lumen may be a blood vessel, as an artery or a vein of a patient's vascular system, including cardiac vasculature, peripheral vasculature, neural vasculature, renal vasculature, and/or or any other suitable lumen inside the body. For example, the imaging device 110 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers, interior of the heart, or other parts of the heart, and/or other systems of the body. In addition to natural structures, the device 110 may be used to examine man-made structures such as, but without limitation, heart valves, stents, shunts, filters and other devices.

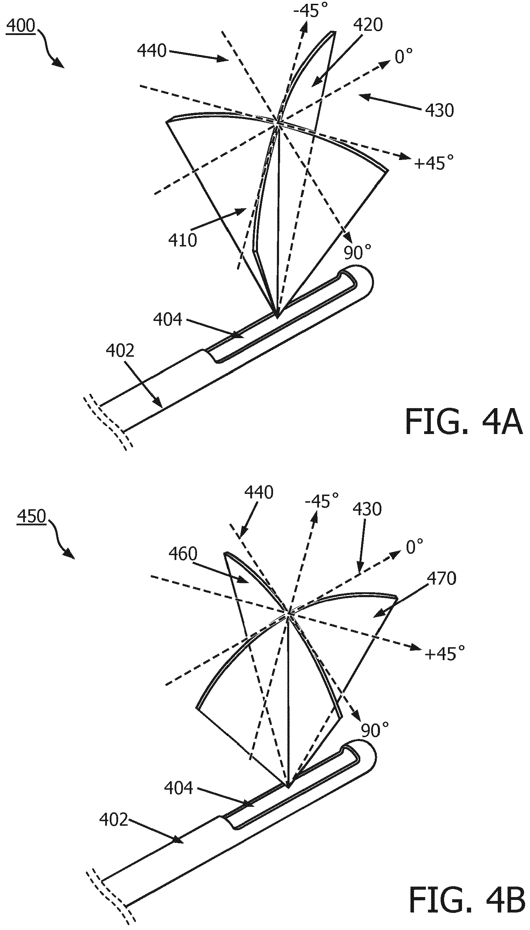

Fig. 4A is a schematic diagram 400 illustrating two imaging planes extending at oblique angles relative to a longitudinal axis of an asymmetric aperture of an imaging array according to aspects of the present disclosure. In particular, Fig. 4A shows imaging planes 410 and 420 extending at +45 degree and -45 degree angles, respectively, relative to a longitudinal axis (also referred to as the axial direction) 430 of the aperture 404. In other embodiments, the imaging planes 410 and 420 may extend at other oblique angles relative to the longitudinal axis 430 and/or a short axis 440 of the aperture 404 that extends perpendicular to the longitudinal axis 430. In some instances, the imaging planes 410 and 420 are at perpendicular angles relative to one another to facilitate x-plane and/or 3D imaging. In some instances, the imaging planes 410 and 420 extend at an oblique angle relative to one another. The diagram 400 shows the catheter tip 402 consistent with the tip 200 in Fig. 2 and also shows the aperture 404 of the array of acoustic elements, which are arranged in a two-dimensional rectangular array. However, the diagram 400 can be representative of any asymmetric aperture of an imaging array for intraluminal imaging.

Fig. 4B is a schematic diagram 450 illustrating two imaging planes extending coplanar with and at a right angle, respectively, relative to a longitudinal axis of an asymmetric aperture of an imaging array. In particular, Fig. 4B shows imaging planes 460 and 470 extending at +90 degree and 0 degree angles, respectively, relative to the longitudinal axis 430 of the aperture 404 of the array of acoustic elements.

In some cases, the resolution of imaging data may decrease for imaging planes with an angular orientation approaching 90 degree with respect to the longitudinal axis 430 of the aperture 404. For example, the resolution of imaging data is noticeably worse but acceptable for imaging planes up to 60 degree, and degrades rapidly from 60 degree to 90 degree. In some examples, significant resolution degradation occurs in an exclusion angle range from 45 degrees to 90 degree. In some other examples, the exclusion angle range is from 60 degree to 90 degree.

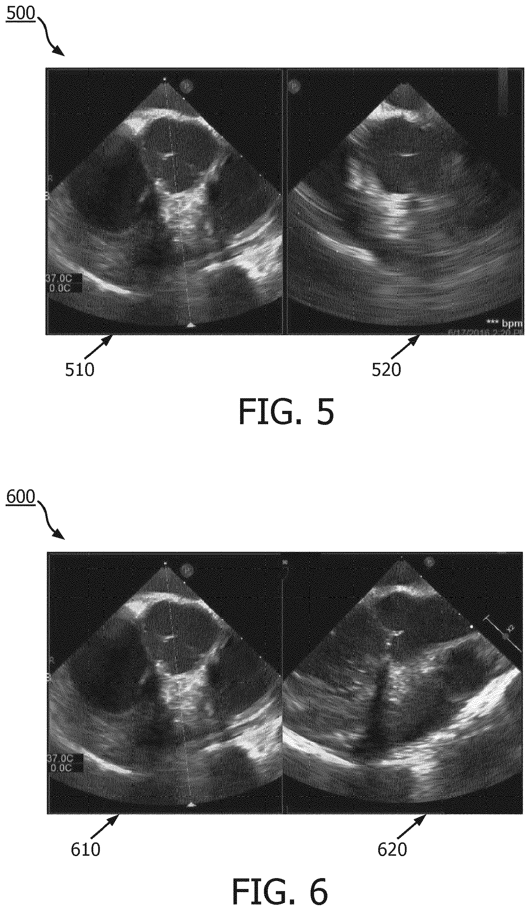

Fig. 5 shows x-plane images at 0 degree and 90 degree angles. The images 500 may be displayed as side by side images from 0 degree and 90 degree views, with the 0 degree view image 510 being on the left. As shown the image 510, the 0 degree view has a better resolution compared with the 90 degree view image 520 on the right.

Fig. 6 shows x-plane images at 0 degree and +45 degree angles. The images 600 may be side by side images from 0 degree and +45 degree views, with the 0 degree view image 610 being on the left. As shown in the example of Fig. 6, the 0 degree view image 610 and the +45 degree view image 620 have comparable resolution performance. In some examples, when displaying one or both of the +45 degree view and the -45 degree view images, one of the 0 degree view or the 90 degree view images is also displayed alongside of them to help a user understand the orientation.

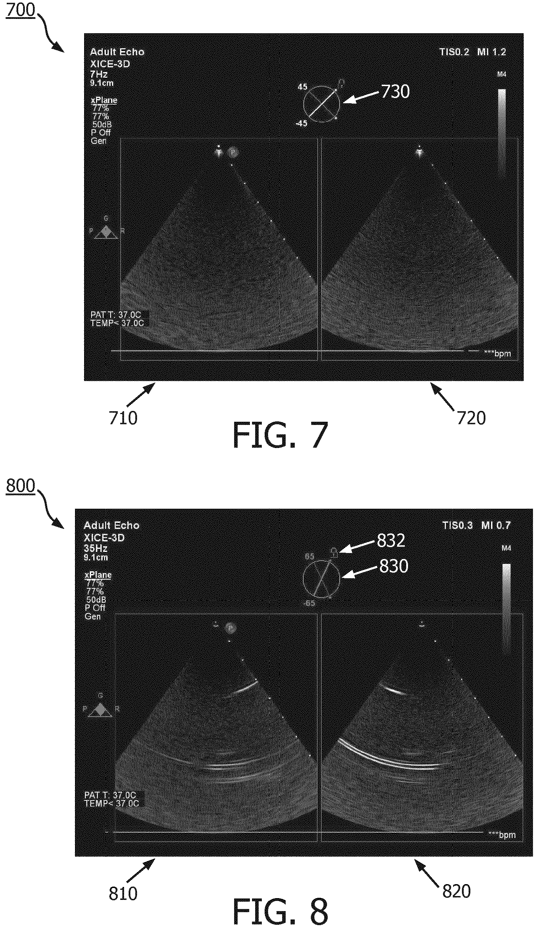

Fig. 7 shows x-plane images at +45 and -45 degree angles on a display 700. The images 710, 720 may be side by side images, with the +45 degree view image 710 being on the left. As shown, the +45 degree view image 710 and the -45 degree view image 720 have comparable resolution performance. The display 700 may include a visual indicator such as an icon or graphic 730 and/or text showing the angles of the image planes used for the images 710, 720. In the example of Fig. 7, a representation of the image planes is shown on the graphic 730 which shows an estimation of the angular distance between the different image planes. This may help a user to understand the imaging data associated with the imaging planes, and may warn a user when this data may include resolution problems. The angular measurements of the imaging planes may be included in the graphic 730.

Fig. 8 shows x-plane images at +65 and -65 degree angles on a display 800. The images 810, 820 may be side by side images, with the +65 degree view image 810 being on the left. As shown, the +65 degree view image 810 and the -65 degree view image 820 have comparable resolution performance. The graphic 830 on the display 800 may notify a user that the imaging data shown in images 810, 820 contains decreased resolution. For example, the color of various portions of the graphic 830 may change depending on the angle of the imaging planes. In the example of Fig. 8, the imaging planes of the graphic 830 are colored red. The color may signify that the imaging planes are oriented beyond ±60 degrees from the axial direction of the transducer. The graphic 830 may include other colors to indicate potential resolution problems at various angles. For example, a first color may be used to show imaging planes at over ±45 degrees, a second color may be used to show imaging planes at over ±60 degrees, and a third color may be used to show imaging planes close to ±90 degrees. In other embodiments, a spectrum ranging from a first color to a second color may be used to show angles approaching ±90 degrees.

In some embodiments, the graphic 830 may include an indicator 832 showing an imaging plane that is locked into place. In this case, another imaging plane may be moved with respect to the locked imaging plane. For example, an imaging plane may be positioned at a predetermined angular distance from the locking imaging plane, such as ±90 degrees, ±60 degrees, ±45 degrees, and other angles. In the example of Fig. 8, the indicator 832 shows that the imaging plane positioned at -65 degree is locked.

The graphic 830 may also be configured to indicate the angle between imaging planes. For example, if the imaging planes used are orthogonal (such as in the example of Fig. 7), the graphic 730 may show a white color or other indication. If the imaging planes are not orthogonal (such as in the example of Fig. 8), the graphic 830 may show a red color or other indication. The graphic 830 may also include other text, colors, textures, highlights, images, or shading to notify the user of the relative angles of imaging planes and possible resolution problems.

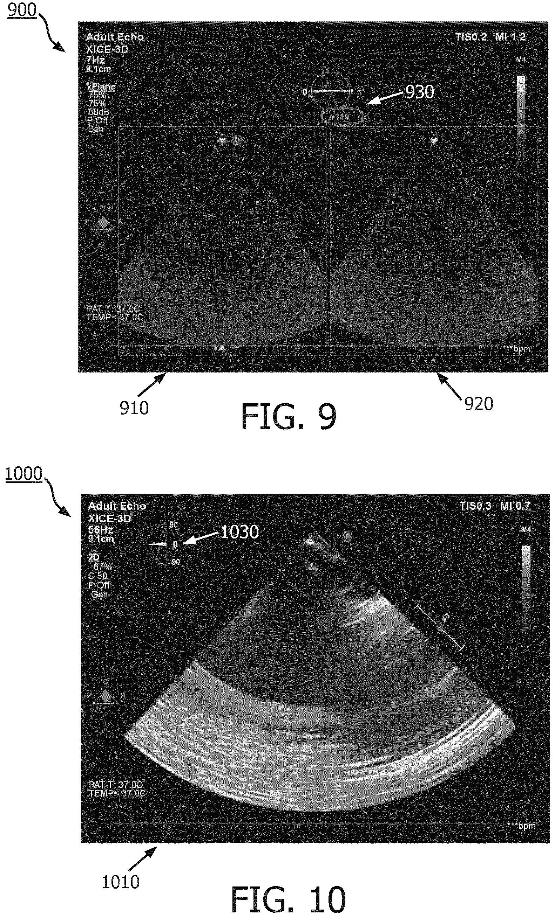

Fig. 9 shows x-plane images at 0 and -110 degree on a display 900. The images 910, 920 are side by side images, with the 0 degree view image 910 being on the left. The graphic 930 on the display 800 may notify a user that the imaging data shown in image 810 (corresponding to the white colored image plane) does not contain resolution problems and that the imaging data shown in imaging 820 (corresponding to the white colored image plane) may contain resolution problems. In the example of Fig. 9, the graphic 930 includes a circle around the text of the -110 degree image plane to further notify a user that resolution problems may occur in the imaging data associated with the -110 degree image plane.

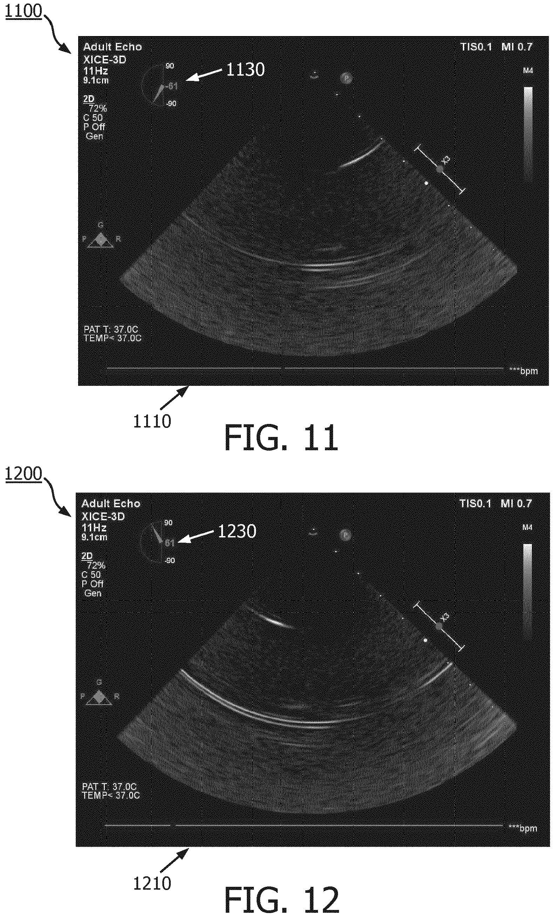

Figs. 10-12 show x-plane displays with an indicator showing the angular orientation of imaging planes. The color and position of the indicator may show levels of possible resolution problems in imaging data associated with the imaging plane. In the example of Fig. 10, the display 1000 includes an indicator 1030 with an imaging plane orientated at 0 degree. The indicator 1030 is colored white and positioned in the center between 90 degrees and -90 degrees, indicating that the image 1010 likely does not include resolution problems. In the example of Fig. 11, the display 1100 includes an indicator 1130 with an imaging plane orientated at -61 degrees. The 1130 indicator is colored red and positioned with a downward orientation, indicating that the image 1110 likely includes resolution problems. In the example of Fig. 12, the display 1200 includes an indicator 1230 with an imaging plane orientated at +61 degrees. The indicator 1230 is colored red and positioned with an upward orientation, indicating that the image 1210 likely includes resolution problems. The indicators 1030, 1130, 1230 may also include other text, colors, textures, highlights, images, or shading to notify the user of the relative angles of imaging planes and possible resolution problems.

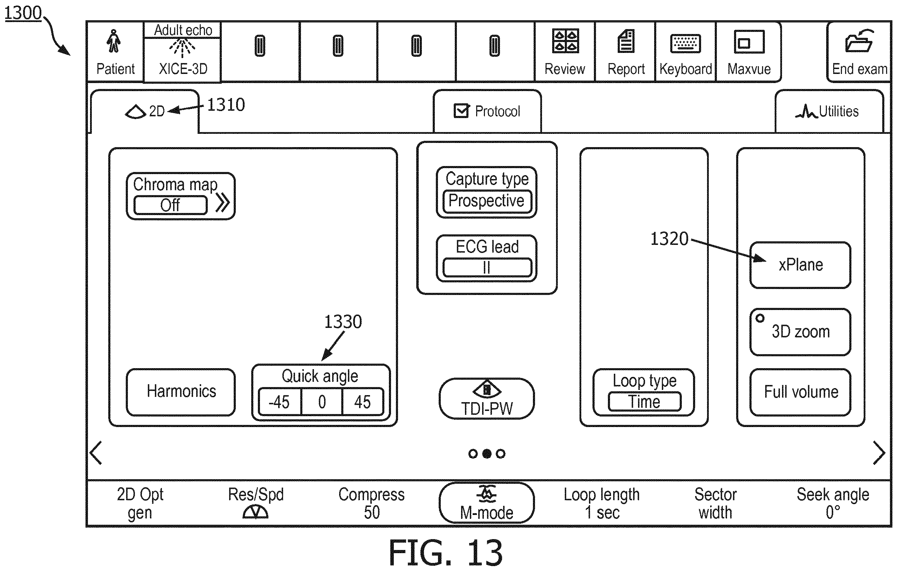

Fig. 13 shows a user interface 1300 for controlling x-plane images. In some embodiments, the user interface 1300 is communicatively connected to the control and processing system 130 as shown in Fig. 1. For example, the user interface 1300 may be electrically or wirelessly connected to the control and processing system 130. The user interface 1300 may be configured to receive selections from a user and send signals to an imaging array. In particular, the user interface 1300 may be used to control the number and orientation of imaging planes. The user interface 1300 may also be used to input, store, and display information about one or more procedures and one or more patients. For example, the user interface 1300 may be used to display one or more images such as those shown in Figs. 5-12. The user interface 1300 may be operated on a computer, a tablet, a Patient Interface Module (PIM), or other type of display device. In some embodiments, the user interface 1300 is a touch screen interface. In other embodiments, the user interface 1300 comprises one or more selectable buttons, switches, toggles, keys, pedals, footswitches, or other input devices. In some embodiments, the user interface 1300 may be used to display imaging data in real time. For example, a user may select options to change the angles of imaging planes and simultaneously view imaging data associated with the imaging planes.

In some embodiments, the user interface 1300 may include a 2D selection area 1310. The 2D selection area may be configured to control aspects of the display relating to a 2D image, such as those shown in Figs. 10-12. The 2D selection area may include one or more selectable options, including a quick angle button 1330. The quick angle button 1330 may be used to automatically orient an imaging plane to a predetermined angle, such as -45 degree, 0 degree, or 45 degree. Other angles may be included in the quick angle button 1330, for example, -60 degree, 60 degree, -90 degree, 90 degree, and other angles. In some embodiments, a user may select an angle on the quick angle button 1330 to orient the imaging plane to the chosen angle by tapping the button once, and reorient the imaging plane to the original angle by tapping the button a second time.

The user interface 1300 may also include an x-plane mode which may be accessed by selection of an xPlane button 1320. The x-plane mode may show two images side by side. In some embodiments, selection of the xPlane button 1320 may automatically compile two 2D images in a side by side configuration, such as shown in Figs. 5-9. For example, the first 2D image may be the 2D image controlled by the 2D selection area 1310 of the user interface 1300. Selecting the xPlane button 1320 may maintain the orientation of the image plane associated with the 2D image in the x-plane display. This may help to minimize disorientation of the user by keeping one of the images constant. A second 2D image may then be added to the x-plane display. In some embodiments, the imaging angles of the first and second imaging planes are predetermined based on a criterion. The predetermined criterion may constitute a relation between the first image and the second image such as a quality or quantity relationship between the first and second image. For example, the criterion can be related to the resolution of the two images. As noted the typical x-plane images at 0 degree and 90 degree may not have a similar resolution when the aperture of the array of acoustic elements 302 is not symmetric, e.g., rather than being square it is rectangular. In some examples, the first angle and the second angle are selected such that the first and second images have comparable resolution and at the same time they are essentially perpendicular to each other, such as +45 degree and -45 degree relative to the longitudinal dimension of the array. In particular, the second 2D image is as close to orthogonal as possible with respect to the first 2D image while avoiding large resolution problems. In particular, the second 2D image may be oriented to avoid regions of resolution problems associated with image planes between 60 and 90 degrees (or -60 and -90 degrees). For example, a first 2D image may be associated with a 0 degree image plane. As a user selects x-plane mode (i.e., pressing the xPlane button 1320), the x-plane image is automatically displayed with a first 2D image associated with the 0 degree image plane and a second 2D image with a 60 degree image plane.

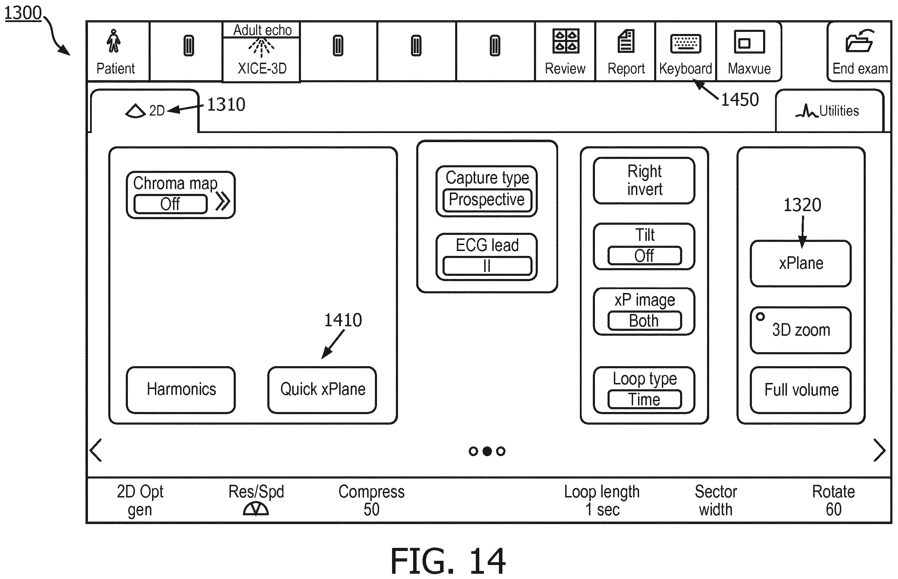

In some embodiments, the orientation of imaging planes may be automatically changed in x-plane mode. For example, the user interface 1300 may be configured to allow a user to automatically change the imaging planes to predetermined angles, such as ±45 degree. In some embodiments, selection of the xPlane button 1320 opens additional options in the 2D selection area. For example, Fig. 14 shows a user interface 1300 including a Quick xPlane button 1410 that may be selected by a user to automatically change the orientation of the imaging planes. Changing the imaging planes automatically to predetermined angles may provide improved image quality. For example, the predetermined orientations may be selected to minimize problems with resolution resulting from image planes in the restricted zone (i.e., from 60 to 90 degrees or -60 to -90 degrees). Furthermore, the predetermined orientations may be selected to be orthogonal (or approaching orthogonal) to provide better imaging data. In some embodiments, the predetermined orientations are +45 and -45 degrees, 0 and 60 degrees, 0 and - 60 degrees, 60 and -60 degrees, and other combinations of angles.

Selecting the Quick xPlane button to automatically change the orientation of imaging planes may save time for the user as well as providing clear results. For example, the automatic change of angles may not require a user to adjust settings of each imaging plane manually, which may require time and experience. Furthermore, since the user is able to select the Quick xPlane button, the user will be aware that the orientation of the imaging planes and associated imaging data will change, thereby avoiding confusion.

Once the imaging planes have been reoriented to the predetermined orientations, the user interface 1300 may be used to make further changes to the orientations of the imaging planes. For example, the user interface 1300 may receive selections from a user to make further adjustments to the imaging planes independently or together (i.e., while maintaining orthogonally of the imaging planes). In some embodiments, the user interface 1300 may be switched from x-plane mode back to 2D mode. In this case, the user interface 1300 may restore the angle of imaging plane (before having changed to x-plane mode).

In some embodiments, the user may make a selection to automatically toggle between various optimizations for imaging data. For example, a user may make a selection on a button, toggle, or switch on the user interface 1300 (such as button 1450 or other button) and/or the intraluminal imaging device 110 (as shown in Fig. 1) to toggle between optimizations for soft biological tissue and interventional devices. The optimizations may be predetermined changes to the aspects of the imaging data, such as one or more of gain, dynamic range, gray map, spatial smoothing, beamforming, frequency, and chroma. In some embodiments, the optimizations may include changes to both gain and gray map, as well as other changes. These optimizations may be made automatically using a predetermined algorithm and without changing unrelated settings such as depth or imaging plane angle. In some embodiments, the user may be able to toggle between the optimizations, as well as the imaging data before optimization. For example, the imaging settings may be automatically saved in memory when the user selects and optimization, such that the user can quickly return to any selected optimization. The ability to quickly select different optimizations may improve image quality and save time. For example, a user desiring to focus on tissue within the imaging data may not be able to clearly see interventional devices such as guidewires, catheters, transducers, stents, and other devices within the imaging data, because these devices may cause reflections and blooming in the imaging data at tissue-specific settings. Similarly, device-specific settings may cause tissue to appear distorted or without sharp details. Therefore, the methods and systems to switch between these various settings may allow a user to easily focus on desired data without having to spend time to manually adjust settings.

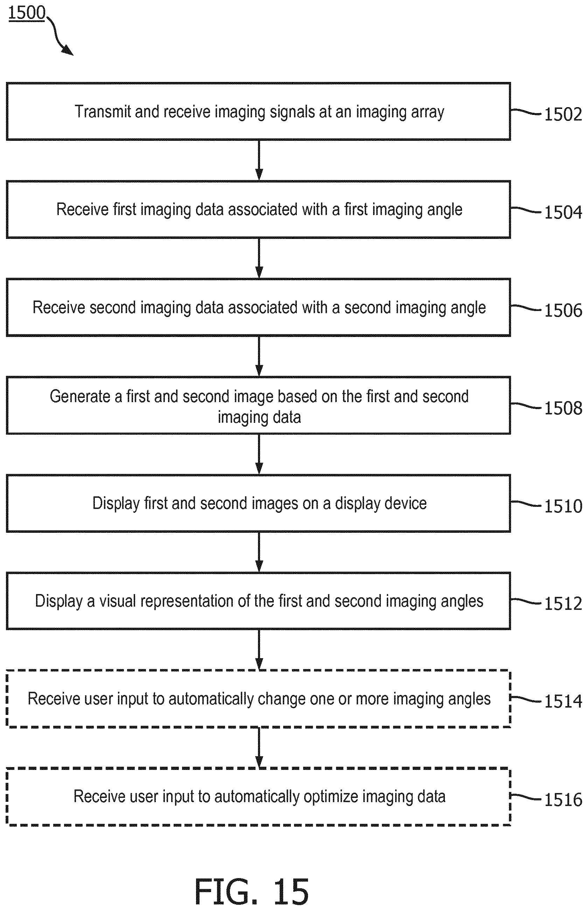

Fig. 15 provides a flow diagram illustrating a method 1500 of intraluminal imaging of a vessel. The method 1500 can be performed with reference to Figs. 1-14. At step 1502, the method may include transmitting and receiving imaging signals at an imaging array. The imaging array may be an ultrasound imaging array. For example, imaging signals may be transmitted and received at a side-looking array of acoustic elements such as the array of acoustic elements 302 as shown in Figs. 2 and 3. The array of acoustic elements 302 may be positioned within the distal portion 104 of an intraluminal imaging device 110. In some examples, a micro-beamformer IC 304 is directly coupled to the array of acoustic elements 302 and transmits and receives imaging signals, e.g., ultrasound signals. In some examples, the array of acoustic elements 302 is an array of ultrasonic transducers. In some embodiments, a connection cable 122 couples the flexible elongate member to a control and processing system 130. The micro-beamformer IC 304 may send the first imaging signals and the second imaging signals through the connection cable 122 to a control and processing system 130 that is configured to construct the first image and the second image. In some embodiments, the control and processing system 130 is configured to send one or more commands including beam forming commands to the micro-beamformer 304.

At step 1504, the method 1500 may include receiving first imaging data associated with a first imaging angle. In some embodiments, the first imaging signals are received by the array of acoustic elements 302 and are beamformed. The beamforming can be performed with reference to Figs. 3 such that the micro-beamformer IC 304 can be coupled, e.g., from beneath, to the array acoustic elements 302 and can provide the required beamforming delays. The micro-beamformer IC 304 can command the array acoustic elements 302 and can transmit and receive signals, e.g., ultrasound signals. The micro-beamformer IC 304 can also include a plurality microchannels delay lines. The micro-beamformer IC 304 can supply the required delays for beamforming from one or more of the microchannels delay lines to the array of acoustic elements 302. In some examples, the beamforming is performed during both transmitting and receiving. In some other examples, the beamforming is performed during the receiving. In some examples, the ultrasound signals received by the acoustic elements are beamformed by applying the required delays to construct a first beam-formed signal associated with a first imaging plane at a first angle relative to an axial direction of an aperture of the side-looking array of acoustic elements.

At step 1506, the method 1500 may include receiving second imaging signals associated with a second imaging angle. In some embodiments, the second imaging signals are received by the array of acoustic elements 302 and are beamformed. As noted, the beamforming can be performed with reference to Figs. 3. The micro-beamformer IC 304 can supply the required delays to provide beamforming for the second imaging signals such that beamforming is provided by applying the required delays to the signals of each of the acoustic elements of the array of acoustic elements 302. In some examples, the ultrasound signals received by the acoustic elements are beamformed by applying the required delays to construct a second beam-formed signal associated with a second plane at a second angle relative to an axial direction of an aperture of the side-looking array of acoustic elements.

At step 1508, the method 1500 may include generating a first and second image based on the first and second imaging data. The first image may be generated from the first imaging signals and the second image may be generated from the second imaging signal, such that the first image corresponds to a view at first plane and the second image corresponds to a view at the second plane. In some embodiments, the first angle is selected as an angle corresponding to an angle of a 2D plane viewed immediately prior to this selection. In some examples, the first angle is manually selected. In some embodiments, the second angle is determined as close as possible to orthogonal to the first angle and additionally avoiding the noted exclusion angle range. In some examples, the first plane and the second plane are essentially at right angles.

At step 1510, the method 1500 may include displaying the first and second images on a display device. For example, the first image can be a graphical/visual representation of the first imaging data and the second image can be a graphical/visual representation of the second imaging data. In some embodiments, the first and second images are displayed side by side in an x-plane mode, such as that shown in Figs. 6-9. The first and second images may be displayed with graphical or textual information such as pointers, axis, labels, and identified objects.

At step 1512, the method 1500 may include displaying a visual representation of the first and second imaging angles. In some embodiments, the imaging angular orientation of the imaging planes is shown in a graphic or indicator with the first and second images. The visual representation of the first and second imaging angles may include an indication of potential resolution problems associated with the first and second imaging angles. For example, the visual representation may notify a user if the first or second imaging angles are more than ±45 degrees, ±60 degrees, or near ±90 degrees. This may help a user better appreciate potential resolution problems resulting from imaging planes with these angles. The indication of potential resolution problems may be represented by a color (i.e., red), a pattern, text, an icon, or other type of graphical indicator. In some embodiments, the first and second imaging angles may be assigned a color along a spectrum indicating their proximity to a restricted zone (i.e., range from 60 to 90 degrees). For example, a first imaging angle of 0 degrees may be displayed with a white color while a second imaging angle of 45 degrees is displayed with pink and a third imaging angle of 80 degrees is displayed with red. Other graphical indicators showing angles near or within a problem area may be used. The graphical indicators and associated colors may be displayed on the images themselves, such as on an indicator, axis, pointer, or cursor.

In some embodiments, the graphical representation of the first and second imaging angles may be shown on the same screen as the first and second images. For example, the graphical representation may be show along an axis or other label on of the first and second images. This may allow a user to easily see this information and adjust the imaging angles accordingly.

At step 1514, the method 1500 may optionally include receiving user input to automatically change one or more of the imaging angles. In some embodiments, this step 1514 may include automatically changing the imaging angles to predetermined values, such as 0, ±45 and ±60 degrees. This change may occur upon selection of a button or switch on a user interface, such as a touch screen. In some embodiments, both first and second imaging angles are changed together to optimize imaging resolution. For example, a user may start with a side by side display of two images include a first imaging plane of 0 degrees and a second imaging plane of 90 degrees. Since the 90 degree imaging plane may have resolution problems, the user may select and option to snap the imaging angles to ±45 degrees to improve resolution performance. The user may then be able to further adjust the imaging planes. In some embodiments, these further adjustments may maintain orthogonally between the first and second imaging planes. In other embodiments, one imaging plane may be locked, or both may be moved independently. After viewing imaging data from the adjusted imaging planes, the user may select an option to restore the original imaging plane angles of 0 and 90 degrees.

At step 1516, the method 1500 may optionally include receiving user input to automatically optimize imaging data. For example, a user may select an option on a user interface to toggle between optimizations for soft biological tissue and interventional devices. Each option may include one or more automatic changes to imaging parameters such as gain, dynamic range, gray map, spatial smoothing, beamforming, frequency, and chroma. This step 1516 may include automatically saving imaging settings for optimized data such that a user can switch between optimizations and view associated imaging data quickly.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ultrasound imaging system, comprising:
an intraluminal ultrasound device configured to be positioned within a body lumen of a patient, the intraluminal ultrasound device comprising a transducer array (302) disposed along a distal portion of a flexible elongate member, wherein the transducer array comprises an asymmetric aperture (404) and is configured to obtain imaging data along one or more imaging planes (410,420); and
a processor (130) in communication with the transducer array, the processor configured to:
receive first imaging data from the transducer array along a first imaging plane (410) at a first angular position with respect to an axial direction of the aperture (404);
compare the first angular position to a threshold angular position above which compromised imaging performance occurs;
output, to a display device (700) in communication with the processor, the first imaging data; and
output, to the display device, a visual representation (730) of the first angular position of the first imaging plane (410) with respect to the axial direction of the aperture (404) and wherein the visual representation is based on the comparison of the first angular position to the threshold angular position.

2. The ultrasound imaging system of claim 1, wherein the visual representation is a comparison of the first angular position and the threshold angular position.

3. The ultrasound imaging system of claim 2, wherein the visual representation comprises an alert if the first angular position exceeds the threshold angular position.

4. The ultrasound imaging system of claim 2, wherein the visual representation comprises a color representing a difference between the first angular position and the threshold angular position.

5. The ultrasound imaging system of claim 1, wherein the processor is configured to optimize the imaging data based on a plurality of imaging settings, wherein the processor is configured to receive a first selection of the plurality of imaging settings from a user and display the imaging data with optimized imaging data corresponding to the selected imaging setting on the display device.

6. The ultrasound imaging system of claim 5, wherein the optimizations comprise at least one of gain, dynamic range, gray map, spatial smoothing, beamforming, frequency, or chroma.

7. The ultrasound imaging system of claim 5, wherein the processor is configured to receive the first selection of the plurality of imaging settings from the display device.

8. The ultrasound imaging system of claim 5, wherein the processor is configured to display optimized imaging data corresponding to a first and a second selection of the plurality of imaging settings on the display device, wherein the display device is configured to toggle between the optimized imaging data corresponding to the first and second selections of the plurality of imaging settings.

9. The ultrasound imaging system of claim 1, wherein the processor is further configured to:
receive second imaging data from the transducer array along a second imaging plane at a second angular position with respect to the axial direction of the aperture; and
output the second imaging data to the display device, for example by showing the first and second imaging data simultaneously in a side by side display.

10. The ultrasound imaging system of claim 9, wherein the processor is further configured to:
receive third imaging data from the transducer array along a third imaging plane at a third angular position with respect to the axial direction of the aperture;
receive fourth imaging data from the transducer array along a fourth imaging plane at a fourth angular position with respect to the axial direction of the aperture; and
output the third and fourth imaging data to the display device.

11. The ultrasound imaging system of claim 10, wherein the processor is configured to receive a second selection to automatically change the display of the first and second imaging data to the third and fourth imaging data.

12. The ultrasound imaging system of claim 11, wherein the third angular position is +45 degrees with respect to the axial direction of the aperture, wherein the fourth angular position is - 45 degrees with respect to the axial direction of the aperture.

13. The ultrasound imaging system of claim 11, wherein the third angular position is 0 degrees with respect to the axial direction of the aperture, wherein the fourth angular position is +60 degrees with respect to the axial direction of the aperture.

14. A non-transitory computer readable medium comprising computer readable instructions stored thereon which when executed by a processor of an ultrasound imaging system cause the ultrasound imaging system to perform the following steps:
receiving, at a processor in communication with an intraluminal ultrasound device positioned within a body lumen of patient, first imaging data from a transducer array of the intraluminal ultrasound device, the transducer array comprising an asymmetric aperture and obtaining imaging data along one or more imaging planes, wherein the first imaging data is obtained along a first imaging plane at a first angular position with respect to an axial direction of the aperture;
comparing, at the processor, the first angular position to a threshold angular position above which compromised imaging performance occurs;
outputting, to a display device in communication with the processor, the first imaging data; and
outputting, to the display device, a visual representation of the first angular position of the first imaging plane with respect to the axial direction of the aperture, and based on the comparison of the first angular position to the threshold angular position, optionally, wherein the visual representation comprises an alert if the first angular position exceeds the threshold angular position.

15. The non-transitory computer readable medium of claim 14, further comprising:
receiving a first selection of a plurality of imaging settings from a user; and
optimizing the imaging data based on the first selection of plurality of imaging settings, wherein outputting the first imaging data comprising displaying, on the display device, optimized imaging data corresponding to the first selection of the plurality of imaging settings.

16. The non-transitory computer readable medium of claim 14, further comprising:
receiving, at the processor, second imaging data from the transducer array along a second imaging plane at a second angular position with respect to the axial direction of the aperture; and
outputting, to the display device, the second imaging data in a side by side display simultaneously with the first imaging data.

## Patentansprüche

1. Ultraschall-Bildgebungssystem, umfassend:
eine intraluminale Ultraschallvorrichtung, die so konfiguriert ist, dass sie in einem Körperlumen eines Patienten positioniert werden kann, wobei die intraluminale Ultraschallvorrichtung eine Wandleranordnung (302) umfasst, die entlang eines distalen Abschnitts eines flexiblen länglichen Elements angeordnet ist, wobei die Wandleranordnung eine asymmetrische Öffnung (404) umfasst und so konfiguriert ist, dass sie Bildgebungsdaten entlang einer oder mehrerer Bildgebungsebenen (410, 420) erhält; und
einen Prozessor (130), der mit der Wandleranordnung in Verbindung steht, wobei der Prozessor konfiguriert ist, zum:
Empfangen erster Bildgebungsdaten von der Wandleranordnung entlang einer ersten Bildgebungsebene (410) in einer ersten Winkelposition in Bezug auf eine axiale Richtung der Öffnung (404);
Vergleichen der ersten Winkelposition mit einer Schwellenwert-Winkelposition, bei deren Überschreitung die Bildgebungsleistung beeinträchtigt wird;
Ausgeben der ersten Bildgebungsdaten an eine Anzeigevorrichtung (700), die mit dem Prozessor verbunden ist; und
Ausgeben einer visuellen Darstellung (730) der ersten Winkelposition der ersten Bildgebungsebene (410) in Bezug auf die axiale Richtung der Öffnung (404) an die Anzeigevorrichtung, wobei die visuelle Darstellung auf dem Vergleich der ersten Winkelposition mit der Schwellenwert-Winkelposition basiert.

2. Ultraschall-Bildgebungssystem nach Anspruch 1, wobei die visuelle Darstellung ein Vergleich der ersten Winkelposition mit der Schwellenwert-Winkelposition ist.

3. Ultraschall-Bildgebungssystem nach Anspruch 2, wobei die visuelle Darstellung eine Warnung umfasst, wenn die erste Winkelposition die Schwellenwert-Winkelposition überschreitet.

4. Ultraschall-Bildgebungssystem nach Anspruch 2, wobei die visuelle Darstellung eine Farbe umfasst, die eine Differenz zwischen der ersten Winkelposition und der Schwellenwert-Winkelposition darstellt.

5. Ultraschall-Bildgebungssystem nach Anspruch 1, wobei der Prozessor so konfiguriert ist, dass er die Bildgebungsdaten auf der Grundlage einer Vielzahl von Bildgebungseinstellungen optimiert, wobei der Prozessor so konfiguriert ist, dass er eine erste Auswahl aus der Vielzahl von Bildgebungseinstellungen von einem Benutzer empfängt und die Bildgebungsdaten mit optimierten Bildgebungsdaten, die der ausgewählten Bildgebungseinstellung entsprechen, auf der Anzeigevorrichtung anzeigt.

6. Ultraschall-Bildgebungssystem nach Anspruch 5, wobei die Optimierungen mindestens eines von Verstärkung, Dynamikbereich, Graukarte, räumliche Glättung, Strahlformung, Frequenz oder Chroma umfassen.

7. Ultraschall-Bildgebungssystem nach Anspruch 5, wobei der Prozessor so konfiguriert ist, dass er die erste Auswahl aus der Vielzahl der Bildgebungseinstellungen von der Anzeigevorrichtung empfängt.

8. Ultraschall-Bildgebungssystem nach Anspruch 5, wobei der Prozessor so konfiguriert ist, dass er optimierte Bildgebungsdaten, die einer ersten und einer zweiten Auswahl aus der Vielzahl von Bildgebungseinstellungen entsprechen, auf der Anzeigevorrichtung anzeigt, wobei die Anzeigevorrichtung so konfiguriert ist, dass sie zwischen den optimierten Bildgebungsdaten, die der ersten und zweiten Auswahl aus der Vielzahl von Bildgebungseinstellungen entsprechen, umschaltet.

9. Ultraschall-Bildgebungssystem nach Anspruch 1, wobei der Prozessor ferner konfiguriert ist, zum:
Empfangen zweiter Bildgebungsdaten von der Wandleranordnung entlang einer zweiten Bildgebungsebene in einer zweiten Winkelposition in Bezug auf die axiale Richtung der Öffnung; und
Ausgeben der zweiten Bildgebungsdaten an die Anzeigevorrichtung, z. B. durch gleichzeitige Anzeige der ersten und zweiten Bildgebungsdaten in einer nebeneinander angeordneten Anzeige.

10. Ultraschall-Bildgebungssystem nach Anspruch 9, wobei der Prozessor ferner konfiguriert ist, zum:
Empfangen dritter Bildgebungsdaten von der Wandleranordnung entlang einer dritten Bildgebungsebene in einer dritten Winkelposition in Bezug auf die axiale Richtung der Öffnung;
Empfangen vierter Bildgebungsdaten von der Wandleranordnung entlang einer vierten Bildgebungsebene in einer vierten Winkelposition in Bezug auf die axiale Richtung der Öffnung; und
Ausgeben der dritten und vierten Bildgebungsdaten an die Anzeigevorrichtung.

11. Ultraschall-Bildgebungssystem nach Anspruch 10, wobei der Prozessor so konfiguriert ist, dass er eine zweite Auswahl empfängt, um die Anzeige der ersten und zweiten Bildgebungsdaten automatisch in die dritten und vierten Bildgebungsdaten zu ändern.

12. Ultraschall-Bildgebungssystem nach Anspruch 11, wobei die dritte Winkelposition +45 Grad in Bezug auf die axiale Richtung der Öffnung beträgt, wobei die vierte Winkelposition -45 Grad in Bezug auf die axiale Richtung der Öffnung beträgt.

13. Ultraschall-Bildgebungssystem nach Anspruch 11, wobei die dritte Winkelposition 0 Grad in Bezug auf die axiale Richtung der Öffnung beträgt, wobei die vierte Winkelposition +60 Grad in Bezug auf die axiale Richtung der Öffnung beträgt.

14. Nicht-transitorisches computerlesbares Medium mit darauf gespeicherten computerlesbaren Anweisungen, die, wenn sie von einem Prozessor eines Ultraschall-Bildgebungssystems ausgeführt werden, das Ultraschall-Bildgebungssystem veranlassen, die folgenden Schritte durchzuführen:
Empfangen von ersten Bildgebungsdaten von einer Wandleranordnung der intraluminalen Ultraschallvorrichtung an einem Prozessor, der mit einer intraluminalen Ultraschallvorrichtung, die in einem Körperlumen eines Patienten positioniert ist, in Verbindung steht, wobei die Wandleranordnung eine asymmetrische Öffnung umfasst und Bildgebungsdaten entlang einer oder mehrerer Bildgebungsebenen erhält, wobei die ersten Bildgebungsdaten entlang einer ersten Bildgebungsebene in einer ersten Winkelposition in Bezug auf eine axiale Richtung der Öffnung erhalten werden;
Vergleichen der ersten Winkelposition bei dem Prozessor mit einer Schwellenwert-Winkelposition, bei deren Überschreitung die Bildgebungsleistung beeinträchtigt wird;
Ausgeben der ersten Bildgebungsdaten an eine mit dem Prozessor verbundene Anzeigevorrichtung; und
Ausgeben einer visuellen Darstellung der ersten Winkelposition der ersten Bildgebungsebene in Bezug auf die axiale Richtung der Öffnung an die Anzeigevorrichtung, und basierend auf dem Vergleich der ersten Winkelposition mit der Schwellenwert-Winkelposition, optional, wobei die visuelle Darstellung eine Warnung umfasst, wenn die erste Winkelposition die Schwellenwert-Winkelposition überschreitet.

15. Nicht-transitorisches computerlesbare Medium nach Anspruch 14, das ferner Folgendes umfasst:
Empfangen einer ersten Auswahl aus einer Vielzahl von Bildgebungseinstellungen von einem Benutzer; und
Optimieren der Bildgebungsdaten auf der Grundlage der ersten Auswahl aus der Vielzahl von Bildgebungseinstellungen, wobei das Ausgeben der ersten Bildgebungsdaten das Anzeigen optimierter Bildgebungsdaten, die der ersten Auswahl aus der Vielzahl von Bildgebungseinstellungen entsprechen, auf der Anzeigevorrichtung umfasst.

16. Nicht-transitorisches computerlesbare Medium nach Anspruch 14, das ferner Folgendes umfasst:
Empfangen von zweiten Bildgebungsdaten von der Wandleranordnung entlang einer zweiten Bildgebungsebene in einer zweiten Winkelposition in Bezug auf die axiale Richtung der Öffnung an dem Prozessor; und
Ausgeben der zweiten Bildgebungsdaten an die Anzeigevorrichtung in einer Nebeneinanderanzeige gleichzeitig mit den ersten Bildgebungsdaten.

## Revendications

1. Système d'imagerie ultrasonore, comprenant:
un dispositif ultrasonore intraluminal configuré pour être positionné à l'intérieur d'un lumen corporel d'un patient, le dispositif ultrasonore intraluminal comprenant un réseau de transducteurs (302) disposé le long d'une partie distale d'un élément allongé flexible, où le réseau de transducteurs comprend une ouverture asymétrique (404) et est configuré pour obtenir des données d'imagerie le long d'un ou plusieurs plans d'imagerie (410, 420); et
un processeur (130) en communication avec le réseau de transducteurs, le processeur configuré pour:
recevoir des premières données d'imagerie provenant du réseau de transducteurs le long d'un premier plan d'imagerie (410) à une première position angulaire par rapport à une direction axiale de l'ouverture (404);
comparer la première position angulaire à une position angulaire de seuil au-dessus de laquelle se produisent des performances d'imagerie compromises;
envoyer, à un dispositif d'affichage (700) en communication avec le processeur, les premières données d'imagerie; et
envoyer, au dispositif d'affichage, une représentation visuelle (730) de la première position angulaire du premier plan d'imagerie (410) par rapport à la direction axiale de l'ouverture (404) et où la représentation visuelle est basée sur la comparaison de la première position angulaire à la position angulaire de seuil.

2. Système d'imagerie ultrasonore selon la revendication 1, dans lequel la représentation visuelle est une comparaison de la première position angulaire et de la position angulaire de seuil.

3. Système d'imagerie ultrasonore selon la revendication 2, dans lequel la représentation visuelle comprend une alerte si la première position angulaire dépasse la position angulaire de seuil.

4. Système d'imagerie ultrasonore selon la revendication 2, dans lequel la représentation visuelle comprend une couleur représentant une différence entre la première position angulaire et la position angulaire de seuil.

5. Système d'imagerie ultrasonore selon la revendication 1, dans lequel le processeur est configuré pour optimiser les données d'imagerie sur la base d'une pluralité de paramètres d'imagerie, où le processeur est configuré pour recevoir une première sélection parmi la pluralité de paramètres d'imagerie d'un utilisateur et afficher les données d'imagerie avec des données d'imagerie optimisées correspondant au paramètre d'imagerie sélectionné sur le dispositif d'affichage.

6. Système d'imagerie ultrasonore selon la revendication 5, dans lequel les optimisations comprennent au moins l'un parmi le gain, la plage dynamique, la carte des gris, le lissage spatial, la formation de faisceau, la fréquence, ou la chrominance.

7. Système d'imagerie ultrasonore selon la revendication 5, dans lequel le processeur est configuré pour recevoir la première sélection parmi la pluralité de paramètres d'imagerie du dispositif d'affichage.

8. Système d'imagerie ultrasonore selon la revendication 5, dans lequel le processeur est configuré pour afficher des données d'imagerie optimisées correspondant à une première et une deuxième sélection parmi la pluralité de paramètres d'imagerie sur le dispositif d'affichage, où le dispositif d'affichage est configuré pour basculer entre les données d'imagerie optimisées correspondant aux première et deuxième sélections parmi la pluralité de paramètres d'imagerie.

9. Système d'imagerie ultrasonore selon la revendication 1, dans lequel le processeur est en outre configuré pour:
recevoir des deuxièmes données d'imagerie provenant du réseau de transducteurs le long d'un deuxième plan d'imagerie à une deuxième position angulaire par rapport à la direction axiale de l'ouverture; et
envoyer les deuxièmes données d'imagerie au dispositif d'affichage, par exemple en affichant simultanément les première et deuxième données d'imagerie dans un affichage côte à côte.

10. Système d'imagerie ultrasonore selon la revendication 9, dans lequel le processeur est en outre configuré pour:
recevoir des troisièmes données d'imagerie provenant du réseau de transducteurs le long d'un troisième plan d'imagerie à une troisième position angulaire par rapport à la direction axiale de l'ouverture;
recevoir des quatrièmes données d'imagerie provenant du réseau de transducteurs le long d'un quatrième plan d'imagerie à une quatrième position angulaire par rapport à la direction axiale de l'ouverture; et
envoyer les troisième et quatrième données d'imagerie au dispositif d'affichage.

11. Système d'imagerie ultrasonore selon la revendication 10, dans lequel le processeur est configuré pour recevoir une deuxième sélection pour changer automatiquement l'affichage des première et deuxième données d'imagerie dans les troisième et quatrième données d'imagerie.

12. Système d'imagerie ultrasonore selon la revendication 11, dans lequel la troisième position angulaire est de +45 degrés par rapport à la direction axiale de l'ouverture, dans lequel la quatrième position angulaire est de -45 degrés par rapport à la direction axiale de l'ouverture.

13. Système d'imagerie ultrasonore selon la revendication 11, dans lequel la troisième position angulaire est de 0 degré par rapport à la direction axiale de l'ouverture, dans lequel la quatrième position angulaire est de +60 degrés par rapport à la direction axiale de l'ouverture.

14. Support non transitoire lisible par ordinateur comprenant des instructions lisibles par ordinateur stockées sur celui-ci qui, lorsqu'elles sont exécutées par un processeur d'un système d'imagerie ultrasonore, amènent le système d'imagerie ultrasonore à effectuer les étapes suivantes consistant à:
recevoir, au niveau d'un processeur en communication avec un dispositif ultrasonore intraluminal positionné à l'intérieur d'un lumen corporel du patient, des premières données d'imagerie provenant d'un réseau de transducteurs du dispositif ultrasonore intraluminal, le réseau de transducteurs comprenant une ouverture asymétrique et obtenir des données d'imagerie le long d'un ou plusieurs plans d'imagerie, où les premières données d'imagerie sont obtenues le long d'un premier plan d'imagerie à une première position angulaire par rapport à une direction axiale de l'ouverture;
comparer, au niveau du processeur, la première position angulaire à une position angulaire de seuil au-dessus de laquelle se produisent des performances d'imagerie compromises;
envoyer, à un dispositif d'affichage en communication avec le processeur, les premières données d'imagerie; et
envoyer, au dispositif d'affichage, une représentation visuelle de la première position angulaire du premier plan d'imagerie par rapport à la direction axiale de l'ouverture, et sur la base de la comparaison de la première position angulaire à la position angulaire de seuil, facultativement, où la représentation visuelle comprend une alerte si la première position angulaire dépasse la position angulaire de seuil.

15. Support non transitoire lisible par ordinateur selon la revendication 14, consistant en outre à:
recevoir une première sélection parmi une pluralité de paramètres d'imagerie provenant d'un utilisateur; et
optimiser les données d'imagerie sur la base de la première sélection parmi une pluralité de paramètres d'imagerie, où l'envoi des premières données d'imagerie comprend l'affichage, sur le dispositif d'affichage, de données d'imagerie optimisées correspondant à la première sélection parmi la pluralité de paramètres d'imagerie.

16. Support non transitoire lisible par ordinateur selon la revendication 14, consistant en outre à:
recevoir, au niveau du processeur, des deuxièmes données d'imagerie provenant du réseau de transducteurs le long d'un deuxième plan d'imagerie à une deuxième position angulaire par rapport à la direction axiale de l'ouverture; et
envoyer, au dispositif d'affichage, les deuxièmes données d'imagerie dans un affichage côte à côte simultanément avec les premières données d'imagerie.
